# EUROPEAN PATENT APPLICATION

(11) **EP 4 059 506 A1**
(43) Date of publication of application: **21.09.2022**
(21) Application number: 22162554.4
(22) Date of filing: 16.03.2022
(51) Int. Cl.: A61K 31/734, A61K 9/00, A61K 31/10, A61K 31/194, A61K 31/728, A61K 36/28, A61K 36/47, A61K 47/20, A61K 47/36, A61P 1/04, A61K 9/06

(54) **COMPOSITION FOR THE TREATMENT OF REFLUX ESOPHAGITIS AND RELATED SYMPTOMS**

(30) Priority: 17.03.2021 IT 202100006338
(71) Applicant: Euro-Pharma S.r.l., 10127 Torino (IT)
(72) Inventor: MISCIOSCIA, Mario, deceased (IT)
(74) Representative: Coppo, Alessandro

(57) **Abstract**

Composition for use in the treatment of acid reflux disease comprising an alginate, such as sodium alginate, methylsulfonylmethane, and an alkali metal salt of a polycarboxylic acid having 2-5 carboxylic groups, preferably potassium citrate.

The composition may further contain hyaluronic acid, in particular in a salt form, an extract of Emblica officinalis and Stevia Rebaudiana.

## Description

### FIELD OF THE INVENTION

The present invention relates to a composition for the treatment of reflux esophagitis and related symptoms.

The present invention originates in the pharmaceutical and nutraceutical fields.

Specifically, the present invention concerns a composition in the form of an alginate-based gel for the treatment of gastroesophageal reflux and/or for the prophylaxis of gastric mucosa and/or esophagus damage caused by the ascent of gastric juices into the esophagus.

### STATE OF THE ART

Gastroesophageal reflux disease is a widely spread disorder affecting approximately 20% of the population in Western countries.

The term gastroesophageal reflux disease, also known as GERD, refers to a complex symptomatology having the ascent of a portion of gastric contents in the esophagus as a pathogenic determinant that causes irritation and inflammation of the esophageal mucosa, and in some cases of the laryngeal mucosa.

The mucous membrane of the stomach is endowed with a protective barrier that prevents acidic juices from damaging it; conversely, the mucous membranes of the esophagus, lacking a protective layer, are potentially exposed to the irritating action of gastric juices. Consequently, the regurgitation of gastric contents, and the possible microaspiration thereof in the upper aerodigestive tracts, brings the esophageal and laryngeal mucosa into contact with the highly acid secretory material of gastric origin. In these circumstances, damage to the affected mucous membranes occurs and a local inflammation process, often accompanied by pain, is established.

In not altered physiological conditions of the human body, the esophageal musculature, thanks to its contractile properties, helps move the food bolus from the jaws to the stomach and prevent its ascent from the stomach.

Two valve muscle devices, the lower and upper esophageal sphincter, LES and UES, respectively, also contribute to this food flow control mechanism. These two sphincters effectively oppose the reflux of gastric secretion from the stomach towards the oral cavity and prevent the reverse progression of the gastric secretion.

In some circumstances, however, this mechanism does not work efficiently, and GERD symptoms occur.

Smoking, alcohol, obesity, hormonal problems such as hyperprolactinemia, stress, age, and the presence of a hiatal hernia are among the main causes that contribute to determining this disease.

In the presence of one or more of these causes, the esophageal mucosa undergoes damage, resulting in reflux esophagitis condition, characterized by retrosternal heartburn and pain, that may be violent and sometimes accompanied by retrosternal spasms and colics.

In cases where gastric reflux is more modest and no overt esophagitis symptoms arise, pharyngeal/laryngeal and pulmonary symptoms may however occur, which are more nuanced and difficult to identify such as recurrent hypopharyngeal and oropharyngeal burns, sialorrhea, hypopharyngeal/laryngeal bolus sensation, sensation of external laryngeal constriction, cough, especially at night and in the supine position, and dysphonia (changes in the tone of the voice).

To manage these conditions, alginate-based preparations for oral administration with a protective action on the mucosa have been formulated.

When they come into contact with acid gastric juices of the stomach, alginate-based formulations form a gelatinous foam or a sort of raft that floats on the gastric contents. When reflux occurs, this "floating" raft ascends through the esophagus first, thus preceding the gastric juices and creating a protective layer on the esophageal mucosa which limits erosion and irritation.

Formulations of this type, which are widely used, contain alginates in combination with antacid substances, such as sodium or potassium bicarbonate or calcium carbonate. The Applicant, while studying the effects of alginate-based preparations for the treatment of gastroesophageal reflux, has found that, despite the treatment, more nuanced esophagitis symptoms remain. These minor symptoms includes hypopharyngeal and oropharyngeal burns, hypopharyngeal/laryngeal bolus sensation, sensation of external laryngeal constriction often associated with cough and coated tongue.

Therefore, there is currently a not fully satisfied demand for products that treat the most overt manifestations of reflux esophagitis while reducing the minor symptoms associated with this disease, in particular inflammation and painful states of esophagus and larynx caused by microaspiration of gastric content at acid pH.

An object of the present invention, therefore, consists in providing an alginate-based composition for the treatment of reflux esophagitis that combines a local anti-inflammatory and pain-relieving action with a protective action on the mucosa of the esophagus and upper aerodigestive tracts.

Another object of the invention consists in providing an alginate-based composition for the treatment of reflux esophagitis whose administration exerts an effect on minor, and more difficult to diagnose, esophagitis symptoms such as mild hypopharyngeal and oropharyngeal burning, hypopharyngeal/laryngeal bolus sensation and/or sensation of external laryngeal constriction, nocturnal cough.

A further object consists in providing an alginate-based gel for oral administration which is free-flowing and easy to take even for the population of subjects affected by gastroesophageal reflux with a local painful component.

### SUMMARY OF THE INVENTION

According to some aspects of the present invention, the inventors have found that by combining an alginate with methylsulfonylmethane and an alkali metal salt of a carboxylic acid with 2-5 carboxylic groups, preferably potassium citrate, a combination of barrier, anti-inflammatory and pain-relieving effects is obtained on the esophagus that allows to reduce symptoms related to reflux disease.

Furthermore, the oral administration of the combination composition described herein increases the speed of healing of esophageal mucosa microlesions and the associated inflammatory state caused by the reflux of gastric material at acid pH.

According to another aspect, the present invention relates to a composition for use in the treatment of acid reflux disease wherein said composition comprises an alginate, methylsulfonylmethane, and an alkali metal salt of a carboxylic acid having 2-5 carboxylic group.

Specifically, the composition is indicated in the combined treatment of reflux gastroesophagitis and associated inflammatory and painful states of the esophagus and upper aerodigestive tracts.

According to some aspects of the invention, the anti-inflammatory, pain-relieving and anti-edema effects of the combination composition are increased by the addition of hyaluronic acid, preferably in the form of a salt.

Advantageously, when taken orally, the composition described herein exerts the double barrier and local anti-inflammatory effect that makes it suitable for the treatment of gastroesophageal reflux and the symptoms that occur following the erosion exerted by gastric juices on the mucosa and/or wall of the esophagus and/or upper aerodigestive tracts.

According to some embodiments, the composition further contains Emblica officinalis also known as Phyllantus emblica, or an extract thereof, and advantageously Stevia Rebaudiana.

Advantageously, the composition according to one of the embodiments described herein finds application both in erosive esophagitis, when erosion of the esophageal mucosa is present, and in non-erosive reflux disease.

Each of the active components contained in the composition of the invention is provided in a pharmaceutically or nutraceutically effective amount.

The composition of the invention may be in a solid or liquid form.

Preferably, the composition described herein is in a liquid or semi-liquid form, preferably of a gel, in particular a soft gel.

According to some embodiments, the composition of the invention is a pharmaceutical composition or a food supplement that can be introduced into the diet of a subject suffering from reflux disease, in particular reflux esophagitis and related symptoms.

### DETAILED DESCRIPTION OF THE INVENTION

The inventors, while carrying out research in the field of gastrointestinal tract diseases, have surprisingly found that by combining an alginate with an organosulfur compound, such as methylsulfonylmethane, and a salt of a carboxylic acid, a double barrier and anti-inflammatory effect is obtained on the mucous membranes of the gastrointestinal or upper aerodigestive tracts, which determines a rapid resolution of the symptoms related to gastroesophageal reflux and a prevention of minor and more difficult to diagnose esophagitis symptoms.

Advantageously, the combination of active ingredients of the composition exerts the dual barrier effect on the esophageal mucosa and repair/healing effect on the mucosa damaged by hydrochloric acid.

According to a preferred embodiment, the composition is in liquid form, in particular a gel or soft gel, for oral administration.

The liquid form of the composition, in particular of a gel, helps to solve the technical problem of providing a viscosity and a consistency which favor the swallowing and flowing along the esophagus, thus limiting the risk of further damaging the esophagus mucosal layer.

In a general aspect, the invention therefore relates to a combination composition for use in the treatment of reflux disease, as defined in the attached claim 1. Embodiments of the composition for uses according to the invention are defined in the attached claims 2-10.

One of the components of the composition of the invention is an alginate, in particular an alkali metal salt of an alginate, in particular a sodium or potassium salt of alginate. The alginate is preferably sodium alginate.

Typically, alginic acid is a polymer structurally comprising D-mannuronic acid and L-glucuronic acid. Alginic acid has a structure similar to pectin and gels upon contact with water and is able to absorb an amount of water equal to 200 times its weight.

In the gastric environment inside the stomach, the alginate reacts with the HCI-based acidic environment, releasing alginic acid which gels in the presence of the water contained in the gastric juice. The formed gel hinders the reflux of stomach contents into the esophagus, and in the event of ascent it also forms a layer that acts as a protective barrier for the esophageal mucosa.

Suitable alginates may have low, medium, or high viscosity grade. High-grade ones are preferred.

Alginates, in particular sodium alginate, with a high degree of viscosity are those for which the viscosity of an aqueous solution at 1% weight/volume is higher than 500 mPa.s., preferably from 500 to 1500 mPa.s., when determined on a Brookfield RVT viscometer using a spindle number 3 at 20 rpm, at 20°C.

Alginates, in particular sodium alginate, of medium grades are those for which the viscosity of an aqueous solution of about 1% weight / volume is in the range from 200 to 1500 mPa.s., when determined with a Brookfield RVT viscometer using a spindle number 3 at 20 rpm, at 20°C.

The composition of the present invention preferably has an alginate and/or alginic acid content of 2 to 90% by weight, preferably 10 to 80% by weight, preferably 25 to 75% by weight, more preferably 30 to 70% by weight with respect to the total weight of the composition.

In certain embodiments, the gel composition may contain from 100 to 1000 mg, 200 to 800mg, 400 to 600mg of alginate, in particular sodium alginate, per dosage unit containing 20 ml of aqueous carrier.

In the composition of the invention, the alginate is used in combination with methylsulfonylmethane, an organosulfur compound known as dimethyl sulfone or MSM, and having the following formula:

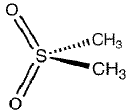

In the composition, this compound has an anti-inflammatory, antioxidant and muscle spasm reduction actions, thus resulting in a relaxation effect of the esophagus and a reduction in the pain associated with the damaging action on the esophageal mucosa due to the regurgitation of gastric juices.

In particular, tests in animal models with gastric lesion induced by ethanol and HCI have shown how MSM performs a gastroprotective action attributable to the ability to inhibit oxidative stress and inflammation. The mechanisms of action involved are diverse and include a reduction in the levels of proinflammatory cytokines TNF-alpha, interleukin (IL-1 beta) and IL-6, chemokine MCP-1 and matrix metalloprotease (MMP)-9, increased levels of glutathione (GSH), catalase (CAT) and prostaglandins E2, reduction in the levels of malondialdehyde (MDA), myeloperoxidase (MPO), carbonyl proteins, and nitric oxide (NO), inhibition of the expression of the NF-kB factor involved in the proinflammatory signaling pathway.

These joint actions result in a local anti-inflammatory action along the entire esophageal mucosa with a reduction in the associated pain and in the healing time of microlesions caused by hydrochloric acid in the case of reflux of gastric contents into the esophagus, which causes irritation and inflammation of the esophageal mucosa.

According to some embodiments, the gel composition may contain methylsulfonylmethane in an amount of 5 to 100 mg, 10 to 60 mg, 20 to 40 mg per dosage unit, in 20 ml of aqueous carrier.

A further component of the composition is a carboxylic acid, preferably an alkali metal salt of a carboxylic acid. Preferably the carboxylic acid is a polycarboxylic acid, preferably having 2-5 carboxylic groups, and in particular 3. Examples of suitable polycarboxylic acids include citric acid, tartaric acid, malic acid, succinic acid, ascorbic acid, adipic acid and fumaric acid. Preferably the (poly)carboxylic acid is citric acid, preferably in the form of a sodium or potassium salt, potassium citrate being the preferred one.

According to some embodiments, the polycarboxylic acid salt as described above, in particular potassium citrate, is contained in the composition in an amount of 5 to 80% by weight, 10 to 70%, 20 to 60%, 30 to 50% by weight with respect to the total weight of the solid components of the composition, i.e. excluding the aqueous carrier, when the composition is in liquid form.

For example, the gel composition may contain from 10 to 700 mg, 20 to 600 mg, 300 to 500 mg of potassium citrate per dosage unit containing 20 ml of aqueous carrier.

Within the scope of the composition described herein, the alkali metal salt of (poly)carboxylic acid, in particular potassium citrate, acts as an antacid, reducing the pH of the gastric environment. For example, when in contact with gastric hydrochloric acid, potassium citrate acts as a base by subtracting hydrogen from hydrochloric acid to generate potassium chloride and citric acid. The latter develops gaseous CO₂ in the gastric environment which maintains the alginate raft in the upper part of the stomach where it can exert its protective action in case of gastroesophageal regurgitation. According to certain embodiments, the composition may contain hyaluronic acid or a physiologically acceptable salt thereof.

Typically, hyaluronic acid is a non-sulphurated glycosaminoglycan with unbranched polysaccharide chain produced by condensation of thousands disaccharide units formed in turn by residues of glucuronic acid and N-acetylglucosamine, linked together, alternatively, by β1→4 and β1→3 glycosidic bonds, as well as by intramolecular hydrogen bonds, which stabilize the conformations thereof.

Preferably the composition contains a hyaluronic acid salt such as sodium, potassium, calcium salt. Sodium salt is preferred.

In some embodiments, hyaluronic acid or physiologically acceptable salt thereof has a molecular weight of 500,000 to 4,200,000 Dalton.

Typically, the molecular weights of hyaluronic acid or salts thereof, as described herein, are expressed as weight average molecular weight (MW) in Daltons.

The average molecular weight of hyaluronic acid can be determined using conventional methods in the art such as those described in Ueno et al., 1988, Chem Pharm Bull. 36, 4971-4975; Wyatt 1993, Anal Chim Acta 272: 1-40; Watt Technologies 1999 "Light scattering University Dawn Course Manual and "Dawn Eos Manual" Wyatt Technology Corp. Santa Barbara CA (USA).

According to some embodiments, the gel composition may contain hyaluronic acid, in particular sodium hyaluronate, in an amount of 1 to 50 mg, 3 to 30 mg, 6 to 15 mg per dosage unit, in 20 ml of aqueous carrier.

The composition may further contain a portion or extract of Phillantus Emblica, also known as Emblica Officinalis. A plant extract for the uses of the invention can be derived from any plant part, such as roots, leaves, fruits, stem or from two or more of these plant parts. Preferably the extract is obtained from Phillantus Emblica fruits.

In some embodiments, the above plant extract, portion or tissue of a Phillantus plant may be present in the composition of the invention in an amount of 0.1 to 60% by weight, 5 to 50% by weight, 20 to 40% by weight, for example 30% by weight. According to some embodiments, the Phillantus Emblica plant extract is obtained by extracting or crushing or grinding a part/portion of the plant, in particular its fruits.

In some preferred embodiments, a suitable Phillantus Emblica plant extract is a dry extract obtained, for example, by grinding the plant matrix or plant portion and drying it according to conventional techniques.

In some embodiments the dry extract has a titer of at least 25% in total tannins (ellagitanning with gallic acid minimum of 8%). For example, a suitable dry extract has a tannin titer of 30%.

Liquid extracts of Phillantus Emblica may be obtained using a physiologically acceptable solvent, such as water, as the extraction medium.

A suitable solvent for obtaining the plant extract is a physiologically acceptable liquid in which the biologically active components are soluble and do not undergo any alterations depriving them of activity.

In some embodiments the solvent is hydrophilic and is selected from water, ethanol, or mixtures thereof.

Additional methods for obtaining Phillantus Emblica plant extract include extraction techniques by digestion, infusion, squeezing, decoction, percolation, counter-current extraction, Soxhlet, supercritical gas or ultrasound extraction.

In certain embodiments, the extraction of one or more biologically active components occurs by maceration of a portion or plant matrix of Phillantus Emblica, preferably fruits, in a suitable solvent, for example water or a hydroalcoholic mixture.

For example, a portion or plant matrix, preferably fruits, of a plant of Phillantus Emblica, is immersed in a suitable solvent, for example a water-ethanol mixture (60-40% v/v), for a suitable time to enrich the solvent with one or more biologically active components.

Under these conditions, the extraction of the biologically active components present in the plant tissues in the solvent occurs by diffusion and osmosis.

Typically, during maceration, the plant matrix of Phillantus Emblica is kept in contact with the solvent for a variable time to obtain the extraction of an effective amount of biologically active component. In certain embodiments, the maceration time can vary between 1 and 48 hours

By way of example, a suitable phytocomplex, obtained by extracting the plant fruits with water - ethyl alcohol (40% by volume of alcohol) as a solvent, contains 445 mg of ascorbic acid/100 g; high density ellagitannins: emblicanin A (37%), emblicanin B (33%), punigluconin (12%), pedunculagin (14%) and, in smaller amounts, Punicafolin and phyllanemblinin A, phyllanemblin; polyphenols, curcumin derivatives.

The presence of a portion or extract of Phillantus Emblica in the composition of the invention helps to regulate the acidity of the gastric environment. Its presence in the composition reduces the frequency and severity of stomach burns and the severity of regurgitation damage on the esophageal mucosa.

In addition, the high content of polyphenols, in particular tannins and flavonoids, acts jointly with MSM to determine the local anti-inflammatory effect and to promote the healing of microlesions of the esophageal mucosa caused by the ascent of HCI from the stomach.

According to some embodiments, the gel composition may contain a Phillantus Emblica extract in an amount of 5 to 200 mg, 10 to 100 mg, 30 to 70 mg per dosage unit, in 20 ml of aqueous carrier.

According to some embodiments, the composition may further contain Stevia Rebaudiana, for example in the form of dry extract obtained according to traditional techniques.

Stevia, in addition to its sweetening action, has a buffering action on stomach acidity, further helping to reduce the symptoms associated with hyperacidity and gastroesophageal reflux.

Stevia Rebaudiana also exerts an anti-hyperglycemic and sweetening action without increasing the risk of raising blood sugar. This effect is particularly useful in the gastric antireflux treatment of diabetic subjects.

Moreover, it was observed that the combination of Emblica Officinalis extract with Stevia Rebaudiana exerts an anti-hyperglycemic activity, helping to maintain glucose homeostasis and improve its metabolism, thus reducing the risk of increasing blood sugar during antireflux therapy. The combination of Emblica Officinalis with Stevia Rebaudiana also helps to boost pancreatic insulin production.

These effects make the embodiments containing Stevia and Emblica Officinalis particularly suitable in the treatment of diabetic subjects suffering from reflux disease. This effect is then particularly appreciable since it has been found that about 20% of the subjects suffering from gastroesophageal reflux are also diabetic or borderline subjects.

According to an embodiment, the composition described herein is a gel containing water, sodium alginate, methylsulfonylmethane (MSM), sodium hyaluronate, dry extract of Emblica officinalis obtained from the fruit having a tannin titer of 30%, potassium citrate, and excipients, for example a stabilizer, preferably glycerol, at least an acidity regulator, in particular sodium lactate and/or citric acid, an aroma, a preservative, in particular potassium sorbate, a sweetener, in particular steviol glycosides obtained from Stevia leaves and/or erythritol.

In the context of the present application, the term combination means that one or more active ingredients are added or mixed with one or other ingredients to give the formulation of the invention. The term combination should therefore not be understood as meaning that the active ingredients are associated with each other with the formation of chemical bonds.

The compositions of the present invention comprise any composition made by administering the combination of active ingredients of the present invention and a physiologically or pharmaceutically acceptable carrier. Such compositions are suitable for food, nutritional, pharmaceutical, or dietary use in mammals, in particular in humans.

According to some embodiments, the composition of the invention is a food supplement or nutritional supplement.

The composition of the invention may assume a wide variety of preparation forms, depending on the desired route of administration.

The compositions for oral administration may be in solid or liquid form.

Preferably the compositions are in fluid or liquid form, in particular of a free-flowing thickened liquid, for example a gel, in particular a soft gel.

Advantageously, the alginate present in the composition in liquid form acts as a thickening biopolymer. The composition can therefore be in the form of a flowing thickened liquid for the uses of the invention.

Thickened liquids are classified in terms of different "consistencies". Guidelines on the classification of thickened fluids consistencies are published in the "National Dysphagia Diet Task Force" (NDDTF), developed by dieticians of the American Dietetic Association (ADA). This publication reports viscosity ranges for each of the three consistency grades: 51-350 cP for the "nectar-like" consistency, 351-1,750 cP for the "honey-like" consistency, and 1,750+ cP for the "thickened spoon" consistency.

The compositions in liquid form contain water as a carrier in which the active components or ingredients are dispersed.

Suitable liquid form compositions for the suppression of gastric reflux comprise a liquid carrier, typically water, containing alginate and the ingredients as previously described. Generally, the liquid compositions of the invention also contain a suspending agent in an amount effective to keep the other ingredients in suspension. The choice of the suspending agent will depend on various factors including the amount and grade of alginate used in the compositions.

According to some embodiments, the liquid compositions contain from 1 to 15% weight/volume of a suspending agent selected from the sodium salt of a cross-linked acrylic polymer with less than 3% by weight of allyl-sucrose, tragacanth gum, pectin, pregelatinized potato starch and sodium starch glycolate, or mixtures of two or more thereof.

Examples of the different types of suspending agents that may be used are the sodium salts of Carbopol 934, 940 and 941 (B F. Goodrich Chemical Company), pectin-apple pectin, pregelatinized potato starch-Prejel 97 (H. Helias & Co, London) and Primojel sodium starch glycolate 110 (Verenigde Zetmeelbdrijven, The Netherlands). Alternatively, the compositions can be in solid form, for example in the form of tablets, lozenges, capsules, powders, granules, pills, chewing gums.

The preparations in solid form may comprise one or more carriers such as, for example, starches, sugars, microcrystalline cellulose, and optionally diluents, granulating agents, lubricants, binders, disintegrating agents.

The tablets, pills, capsules, granules may also contain a binder such as tragacanth gum, acacia, corn starch or gelatin; excipients such as dicalcium phosphate; a disintegrating agent such as corn starch, potato starch, alginic acid; a lubricant such as magnesium stearate; a sweetening agent such as sucrose, lactose, or saccharin and/or Stevia Rebaudiana. If desired, the tablets may be coated using traditional techniques. When the pharmaceutical unit form is a capsule, it may contain a liquid carrier such as a fatty oil, in addition to the materials of the above type.

Various other materials may be present as coatings or to modify the physical form of the pharmaceutical unit. For example, tablets, lozenges may be coated with shellac and/or contain sugar, stevia, or both.

Flavoring agents, preservatives, coloring agents, and the like, may also be present in the composition.

The compositions of the invention are prepared according to conventional pharmaceutical or nutraceutical production techniques, for example by mixing the active ingredients with suitable excipients and/or physiologically acceptable carriers to obtain the desired form.

The compositions for pharmaceutical or nutritional use may be formulated as a single form or dosage unit. In some embodiments, in the compositions of the present invention, the active ingredients are formulated in dosage units.

The dosage unit may contain from 0.1 to 1,000 mg of each active ingredient or plant extract containing the active ingredient per dosage unit for daily administration.

In certain embodiments, the composition of the invention is in liquid or semi-liquid form, for example a gel, which contains 5 to 40 ml, 10 to 30, preferably 15 to 25 ml of liquid, per dosage unit.

Preferably, the single dosage unit is a stick pack containing gel, for example 20 ml per unit.

The term "carrier", as used herein, refers to a medium, excipient, diluent with which the combination of therapeutic or active ingredients is administered.

Any carrier and/or excipient suitable for the form of preparation desired for administration to humans is contemplated for use with the compounds disclosed in the present invention.

A physiologically acceptable or edible carrier may be a pharmaceutically acceptable carrier.

The term "physiologically acceptable" means a physiologically acceptable solid or liquid substance that can be assimilated by the human body, whose intake by humans is approved by the relevant authorities, such as EFSA or EMA or FDA, for use in pharmaceutical, nutritional, or food applications.

The composition described herein is used in the treatment of an acid reflux disease, in particular esophageal reflux, and can also be used for diabetic subjects.

The term *"acid reflux disease"* refers to any disease or condition characterized by stomach contents ascending into the esophagus (esophageal reflux) and/or into the upper digestive or aerodigestive tracts including gastritis, dyspepsia, peptic ulcer, heartburn, extra esophageal gastric reflux, possibly with symptoms or complications, including retrosternal or chest pain, acid taste in the back of the mouth, stomach burn, bad breath, non-cardiac chest pain, vomiting, belching, dyspepsia, esophageal stricture, Barrett's esophagus, pharyngeal/laryngeal and pulmonary symptoms such as hypopharyngeal and oropharyngeal burns, sialorrhea, hypopharyngeal/laryngeal bolus sensation, sensation of external laryngeal constriction, cough and dysphonia.

The composition, in particular the gel for uses according to the invention, may be administered orally to a subject in need of treatment, for example with a frequency of 2 or 3 administrations per day. Preferably, a dosage unit is administered, for example in the form of a sachet containing 20 mg of gel, after meals and/or in the evening before going to sleep.

Some examples of embodiment of the invention are reported below.

### EXAMPLE 1

Composition in the form of a free-flowing gel for the treatment of reflux esophagitis packaged in a single dosage unit containing the following ingredients:

| | |
|---|---|
| Sodium alginate | 500 mg |
| Potassium citrate | 400 mg |
| Emblica dry extract (tannin titer of 30%) | 50 mg |
| Methylsulfonylmethane | 30 mg |
| Sodium hyaluronate | 11 mg |
| Water | 20 ml |

### EXAMPLE 2

Gel for the treatment of reflux esophagitis in stick and/or heat-sealed sachet, especially for diabetics, containing:

| | |
|---|---|
| Sodium alginate | 500 mg |
| Potassium citrate | 400 mg |
| Emblica dry extract (tannin titer of 30%) | 50 mg |
| Methylsulfonylmethane | 30 mg |
| Stevia Rebaudiana | 15 mg |
| Sodium hyaluronate | 11 mg |
| Water | 10 or 20 ml |

Excipients: erythritol, glycerol, sodium lactate, potassium sorbate.

### EXAMPLE 3

Clinical tests related to the oral administration of the gel of Example 2 in the treatment of reflux esophagitis.

30 patients diagnosed with type A esophagitis according to Los Angeles participated in the clinical trial. The diagnosis was made following gastroscopy with sedation.

A first group of 25 patients was treated with the gel of Example 2.

The treatment involved taking a dosage unit of 20 ml gel contained in a heat-sealed bag twice a day. The protocol provided for the administration of the dosage unit in the morning after breakfast and after dinner for thirty days. There was an interval of 10-14 hours between the two daily administrations.

The second group of 5 patients (control group) was treated with a gel containing 2% glucose syrup and the same excipients as the gel of Example 2.

Both patient groups were provided with a questionnaire with questions on the evolution of symptoms related to reflux (stomach burn, presence of nocturnal regurgitation, esophagus and/or throat burns, dry mouth, belching, swollen throat) and on the remission of symptoms. They were asked to report any side effects encountered during treatment.

The result referable to the first group of 25 patients treated with the gel of Example 2 showed a compliance to treatment of 98%. All patients in this group at the end of 30 days of treatment reported a reduction in reflux symptoms, in particular heartburn, burning in the stomach and along the esophagus, cough, and foreign body sensation) after thirty days there was a significant improvement.

None of the 25 patients reported having taken more dosage units than the two required by the therapeutic protocol.

In the group of five patients treated with placebo, in four cases the symptoms remained substantially unchanged throughout the treatment. One patient reported modest improvement of the initial clinical picture.

The results of these clinical trials show and experimentally support the therapeutic efficacy in the treatment of gastroesophageal reflux disease of the gel composition described herein.

## Claims

1. A composition for use in the prevention and/or treatment of acid reflux disease comprising an alginate, methylsulfonylmethane, and an alkali metal salt of a carboxylic acid having 2-5 carboxylic groups, and a pharmaceutically acceptable carrier.

2. The composition for use according to claim 1, wherein the alginate is an alkali metal salt of alginate, preferably sodium or potassium alginate, more preferably sodium alginate.

3. The composition for use according to claim 1 or 2, wherein the alkali metal salt of a carboxylic acid having 2-5 carboxylic group is a sodium or potassium salt of citric acid, in particular potassium citrate.

4. The composition for use according to any one of claims 1-3, further comprising hyaluronic acid or a pharmaceutically acceptable salt thereof, in particular sodium or potassium salt, preferably sodium hyaluronate.

5. The composition for use according to any one of claims 1-4, further comprising Phillantus Emblica or an extract thereof, preferably obtained from fruits, preferably having a tannin titer of at least 25%.

6. The composition for use according to any one of claims 1-5, further comprising Stevia Rebaudiana, in particular for use in the treatment of a diabetic subject or a subject with glycemia higher than physiological values.

7. The composition for use according to any one of claims 1-6, in the form of a water-based gel, preferably a free-flowing soft gel.

8. The composition for use according to any one of claims 1-6, for protecting the esophageal mucosa and repairing or healing the esophageal mucosa damaged by hydrochloric acid.

9. A water-based gel comprising the composition for use according to any one of claims 1-8, comprising sodium alginate, potassium citrate, Phillantus emblica, in particular in the form of a dry extract, methylsulfonylmethane, sodium hyaluronate and a pharmaceutically acceptable, water-based carrier.

10. The gel for use according to claim 9, comprising 500 mg of sodium alginate, 400 mg of potassium citrate, 50 mg of Phillantus emblica dry extract, 30 mg of methylsulfonylmethane, 15 mg of Stevia, 11 mg of sodium hyaluronate and 20 mL of a water-based carrier.
